# FASCICULE DE BREVET EUROPEEN

(11) **EP 0 815 807 B1**
(45) Date de publication et mention de la délivrance du brevet: **28.11.2001**
(21) Numéro de dépôt: 97401391.4
(22) Date de dépôt: 18.06.1997
(51) Int. Cl.: A61F 2/20

(54) **Prothèse intralaryngée**
Kehlkopf-Endoprothese
Laryngeal endoprosthesis

(30) Priorité: 26.06.1996 FR 9607921
(43) Date de publication de la demande: 07.01.1998
(73) Titulaire: Novatech SA, 06130 Grasse le Plan (FR)
(72) Inventeur: Debry, Christian Max, 75015 Paris (FR)
(74) Mandataire: Bonnetat, Christian

(56) Documents cités:
- EP-A- 0 279 484
- WO-A-89/07916
- FR-A- 2 509 605
- US-A- 4 794 924

## Description

La présente invention concerne une prothèse intralaryngée.

On sait que, à la déglutition, la fonction principale du larynx est d'obturer les voies respiratoires pour les protéger et éviter que le bol alimentaire n'y pénètre, au lieu de passer par l'oesophage. Cette obturation se fait par réflexe neuro-musculaire de fermeture de la glotte, résultant de l'adduction de groupes musculaires disposés sur trois niveaux (ary-épiglottique, bandes ventriculaires et cordes vocales). En cas de dysfonctionnement à l'un de ces niveaux, il s'ensuit des troubles de la déglutition, le larynx ne pouvant plus assurer une fermeture correcte des voies respiratoires, de sorte que le bol alimentaire peut passer dans celles-ci. Il en résulte bien entendu des conséquences dramatiques pour le patient.

Jusqu'à présent, seule une trachéotomie permet d'éviter les conséquences dramatiques d'un dysfonctionnement du larynx. Elle implique la mise en place permanente d'un ballonnet trachéal gonflé obturant la trachée en amont d'une canule disposée dans l'orifice de trachéotomie.

On voit donc que la thérapeutique actuelle d'un dysfonctionnement laryngien est particulièrement lourde et mutilante et a des conséquences de très grand inconfort pour les patients. De plus, le ballonnet trachéal risque d'entraîner des complications trachéales, telles que nécrose ou sténose secondaire.

La présente invention a donc pour objet de remédier à ces inconvénients et de permettre de soigner un dysfonctionnement laryngien soit sans aucune trachéotomie, soit avec trachéotomie mais sans ballonnet trachéal, ce qui, dans les deux cas, accroît le confort du patient.

A cette fin, selon l'invention, la prothèse destinée à remédier au dysfonctionnement du larynx est remarquable en ce qu'elle comporte un corps tubulaire destiné à être introduit dans le larynx et comportant, à l'une de ses extrémités conformée en biseau, d'une part, une face d'obturation inclinée et, d'autre part, des orifices latéraux de communication entre l'intérieur et l'extérieur dudit corps tubulaire.

Ainsi, lorsque la prothèse conforme à la présente invention est en position intralaryngée, ladite face d'obturation inclinée dirige le bol alimentaire de la bouche vers l'oesophage, sans possibilité d'introduction dans les voies respiratoires. En revanche, grâce aux orifices latéraux de communication, la circulation d'air n'est pas interrompue dans lesdites voies respiratoires.

Le corps tubulaire peut- être souple, semi-rigide ou même rigide.

De préférence, ladite prothèse est réalisée en une matière biocompatible, par exemple en silicone.

Selon un premier mode de réalisation, ladite face d'obturation inclinée est fixe et solidaire du bord périphérique de l'extrémité biseautée.

Cependant, selon une variante de réalisation, ladite face d'obturation inclinée n'est solidaire de ladite extrémité biseautée qu'à la partie extrême dudit biseau.

Ainsi, dans ce dernier cas, ladite face d'obturation inclinée est battante et forme une sorte de clapet qui obture plus ou moins ledit corps tubulaire, en fonction de l'inspiration, de l'expiration et/ou de la déglutition.

Avantageusement, ledit corps tubulaire comporte des tétons extérieurs saillants, permettant de fixer ladite prothèse en position à l'intérieur du larynx, par appui desdits tétons contre la paroi interne de celui-ci. Avantageusement, pour permettre à ladite prothèse de se loger aisément au niveau des cordes vocales, l'extrémité dudit corps tubulaire, opposée au biseau, présente un diamètre plus faible que du côté dudit biseau.

Avantageusement, lesdits orifices latéraux de communication sont formés par des échancrures dans le bord périphérique de l'extrémité biseautée dudit corps tubulaire.

On remarquera que, par le document FR-A-2 509 605, on connaît déjà une prothèse totale destinée à remplacer intégralement le larynx tout en assurant une déglutition et une respiration par voie haute. Cette prothèse connue constitue donc un larynx artificiel nécessitant obligatoirement une laryngectomie et une trachéotomie.

Ce larynx artificiel connu comporte, à sa partie supérieure, un couvercle de forme générale conique percé d'un orifice auquel est associé un clapet.

Ainsi, une telle prothèse, d'une part, n'est pas appropriée à la thérapeutique des dysfonctionnements laryngés, mais, d'autre part, est obligatoirement constituée d'une pluralité de pièces différentes, réalisées en des matières différentes.

En revanche, la prothèse intralaryngée conforme à la présente invention peut être réalisée en seulement deux pièces, l'une étant le corps tubulaire et l'autre la face d'obturation, en un même matériau biocompatible et collées l'une à l'autre, ou bien encore en une seule pièce de matériau biocompatible incluant le corps tubulaire et la face d'obturation.

Les figures du dessin annexé feront bien comprendre comment l'invention peut être réalisée. Sur ces figurés, des références identiques désignent des éléments semblables.

La figure 1 est une vue latérale d'un premier mode de réalisation de la prothèse intralaryngée conforme à la présente invention.

La figure 2 est une vue en coupe, selon la ligne II-II de la figure 1.

La figure 3 est une vue de dessus, suivant la flèche III de la figure 1.

La figure 4 est une vue latérale d'une variante de réalisation de la prothèse conforme à la présente invention.

La figure 5 illustre la mise en place de la prothèse conforme à l'invention à l'intérieur du larynx d'un patient.

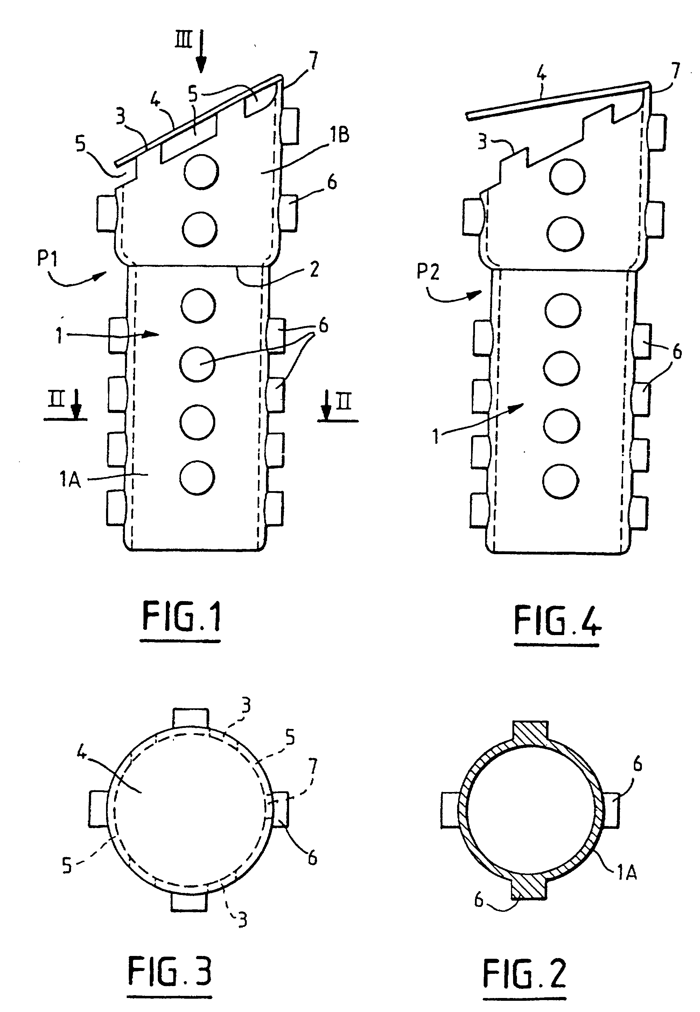

L'exemple de réalisation P1 de la prothèse intralaryngée, conforme à la présente invention et représentée sur les figures 1, 2 et 3, comporte un corps tubulaire 1, dont la partie inférieure 1A présente un diamètre plus petit que la partie supérieure 1B, lesdites parties 1A et 1B étant reliées l'une à l'autre le long d'une ligne 2.

Par ailleurs, l'extrémité de la partie supérieure 1B, opposée à la partie inférieure 1A, est conformée en un biseau 3 et est obturée par une face d'obturation inclinée 4 solidaire du bord périphérique dudit biseau 3.

Dans ce dernier, sont découpées des échancrures 5 assurant la libre communication gazeuse entre l'intérieur et l'extérieur du corps tubulaire 1, au niveau de la partie supérieure de la prothèse. Sur la face extérieure du corps tubulaire 1, sont prévus des tétons saillants 6.

La prothèse P1 peut être souple et réalisée en deux pièces de silicone collées l'une à l'autre par une colle appropriée, à savoir le corps tubulaire 1 et la face d'obturation inclinée 4. Elle peut également être réalisée en une seule pièce de silicone comportant ledit corps tubulaire souple 1 et ladite face d'obturation inclinée 4.

Pour l'essentiel, la variante de réalisation P2 de la figure 4 est identique au mode de réalisation P1 des figures 1 à 3. Cependant, dans le cas de la prothèse P2, la face d'obturation inclinée 4 n'est pas solidaire de la totalité du bord périphérique du biseau 3, mais seulement de la partie extrême 7 dudit biseau 3.

Ainsi, dans ce cas, la face inclinée 4 est battante et peut soit venir s'appliquer contre le bord périphérique du biseau 3, soit s'en écarter.

Comme mentionné précédemment à propos du mode de réalisation P1, le mode de réalisation P2 peut être réalisé en une ou en deux pièces de silicone.

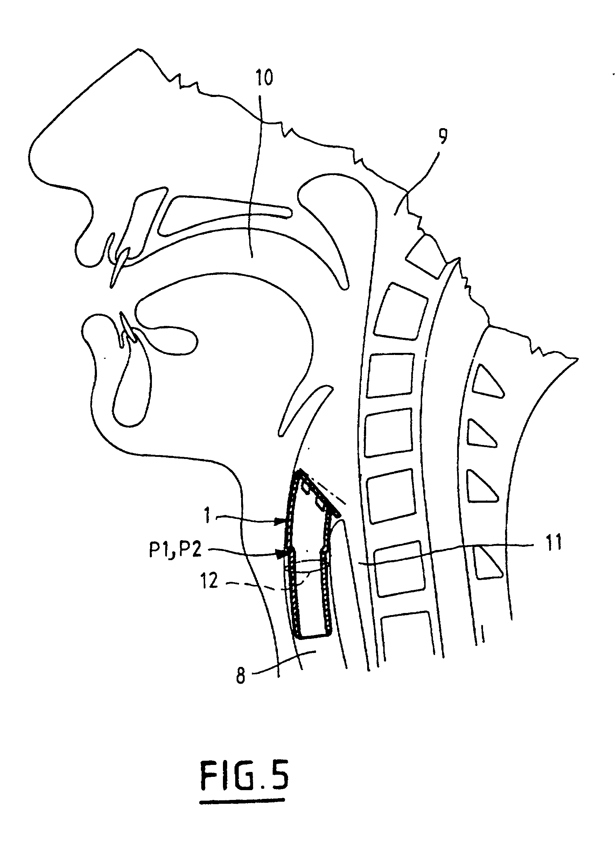

En examinant la figure 5, sur laquelle on a représenté la prothèse P1 ou P2 de l'invention en place dans le larynx 8 d'un patient 9 présentant un dysfonctionnement dudit larynx, on comprend aisément que le bol alimentaire provenant de la bouche 10 dudit patient 9 ne peut pénétrer dans les voies respiratoires de celui-ci à travers le larynx. En effet, la face d'obturation inclinée 4 obture alors le corps tubulaire 1 et dirige ce bol alimentaire vers l'oesophage 11.

En revanche, la prothèse P1, P2 conforme à la présente invention assure en continu la communication aérienne à l'intérieur des voies respiratoires, par l'intermédiaire du corps tubulaire 1, des échancrures 5 et, éventuellement, à travers l'extrémité ouverte du biseau 3 (dans le cas du mode de réalisation P2).

Bien entendu, le diamètre de la partie supérieure 1B du corps tubulaire 1 est adapté à la section de la partie supérieure du larynx 8, tandis que le diamètre de la partie inférieure 1A dudit corps tubulaire 1 est plus petit que celui de la partie supérieure 1B, de façon que cette partie inférieure 1A traverse aisément les cordes vocales 12. Les tétons saillants 6 assurent l'accrochage de la prothèse sur la paroi interne du larynx 8.

## Revendications

1. *Prothèse laryngée (P1, P2) comportant un corps tubulaire (1), qui est destiné à être introduit dans le larynx (8) et dont une extrémité (3) est pourvue d'une face d'obturation inclinée (4),*
***caractérisée en ce que** :*
- *ladite extrémité (3) pourvue de ladite face d'obturation inclinée (4) est conformée en biseau ; et*
- *ladite extrémité biseautée (3) comporte des orifices latéraux (5) de communication entre l'intérieur et l'extérieur dudit corps tubulaire (1).*

2. Prothèse selon la revendication 1,
**caractérisée en ce que** ladite face d'obturation inclinée (4) est fixe et est solidaire du bord périphérique de l'extrémité biseautée (3).

3. Prothèse selon la revendication 1,
**caractérisée en ce que** ladite face d'obturation inclinée (4) est battante et n'est solidaire que de la partie extrême (7) de l'extrémité biseautée (3).

4. Prothèse selon l'une quelconque des revendications 1 à 3,
**caractérisée en ce qu'**elle est réalisée en deux pièces de matière biocompatible assemblées, à savoir ledit corps tubulaire (1) et ladite face d'obturation inclinée (4).

5. Prothèse selon l'une quelconque des revendications 1 à 3,
**caractérisée en ce qu'**elle est réalisée en une seule pièce de matière biocompatible, formant ledit corps tubulaire souple (1) et ladite face d'obturation inclinée (4).

6. Prothèse selon l'une quelconque des revendications 1 à 5,
**caractérisée en ce que** ladite prothèse est réalisée en silicone.

7. Prothèse selon l'une quelconque des revendications 1 à 6,
**caractérisée en ce que** lesdits orifices latéraux (5) sont formés par des échancrures dans le bord périphérique de l'extrémité biseautée (3).

8. Prothèse selon l'une quelconque des revendications 1 à 7,
**caractérisée en ce que** ledit corps tubulaire (1) comporte des tétons extérieurs saillants (6) pour le maintien de ladite prothèse à l'intérieur du larynx.

9. Prothèse selon l'une quelconque des revendications 1 à 8,
**caractérisée en ce que** ledit corps tubulaire (1) présente, au niveau des cordes vocales, un diamètre inférieur à celui qu'il a du côté de ladite extrémité (3) conformée en biseau.

## Claims

1. A larynx prosthesis (P1, P2) including a tubular body (1) which is intended to be introduced into the larynx (8) and one end (3) of which is provided with an inclined closure face (4),
**characterized in that**:
- said end (3) provided with said inclined closure face (4) is shaped in a bevel; and
- said beveled end (3) comprises lateral orifices (5) for communication between the inside and the outside of said tubular body (1).

2. The prosthesis as claimed in claim 1,
**characterized in that** said inclined closure face (4) is fixed and is integral with the peripheral edge of the beveled end (3).

3. The prosthesis as claimed in claim 1,
**characterized in that** said inclined closure face (4) is hinged and is integral only with the extreme portion (7) of the beveled end (3).

4. The prosthesis as claimed in any one of claims 1 to 3,
**characterized in that** it consists of two pieces which are made of biocompatible material and are joined together, namely said tubular body (1) and said inclined closure face (4).

5. The prosthesis as claimed in any one of claims 1 to 3,
**characterized in that** it consists of one single piece made of biocompatible material, forming said flexible tubular body (1) and said inclined closure face (4).

6. The prosthesis as claimed in any one of claims 1 to 5,
**characterized in that** said prosthesis is made of silicone.

7. The prosthesis as claimed in any one of claims 1 to 6,
**characterized in that** said lateral orifices (5) are formed by indentations in the peripheral edge of the beveled end (3).

8. The prosthesis as claimed in any one of claims 1 to 7,
**characterized in that** said tubular body (1) includes external projecting studs (6) for holding said prosthesis inside the larynx.

9. The prosthesis as claimed in any one of claims 1 to 8,
**characterized in that** said tubular body (1) has, at the level of the vocal cords, a diameter which is smaller than the diameter it has at said beveled end (3).

## Patentansprüche

1. Kehlkopfprothese (P1, P2), die einen röhrenförmigen Körper (1) aufweist, der dafür vorgesehen ist, in den Kehlkopf (8) eingeführt zu werden und dessen eine Ende (3) mit einer geneigten Verschlussfläche (4) versehen ist,
**dadurch gekennzeichnet, dass**:
- das mit der geneigten Verschlussfläche (4) versehene Ende (3) mit einer Abschrägung ausgestaltet ist; und
- das abgeschrägte Ende (3) seitliche Öffnungen (5) zur Verbindung zwischen dem Inneren und dem Äußeren des röhrenförmigen Körpers (1) aufweist.

2. Prothese nach Anspruch 1, **dadurch gekennzeichnet, dass** die geneigte Verschlussfläche (4) feststehend und mit einem Umfangsrand des abgeschrägten Endes (3) fest verbunden ist.

3. Prothese nach Anspruch 1, **dadurch gekennzeichnet, dass** die geneigte Verschlussfläche (4) von selbst zufallend und nur mit dem äußersten Teil (7) des abgeschrägten Endes (3) fest verbunden ist.

4. Prothese nach einem der Ansprüche 1 bis 3, **dadurch gekennzeichnet, dass** sie als zwei zusammengesetzte Teile aus biokompatiblem Material, nämlich dem röhrenförmigen Körper (1) und der geneigten Verschlussfläche (4), ausgeführt ist.

5. Prothese nach einem der Ansprüche 1 bis 3, **dadurch gekennzeichnet, dass** sie als ein einziges Teil aus biokompatiblem Material ausgeführt ist, das den röhrenförmigen Körper (1) und die geneigte Verschlussfläche (4) umfasst.

6. Prothese nach einem der Ansprüche 1 bis 5, **dadurch gekennzeichnet, dass** die Prothese aus Silicon besteht.

7. Prothese nach einem der Ansprüche 1 bis 6, **dadurch gekennzeichnet, dass** die seitlichen Öffnungen (5) aus Ausnehmungen vom Umfangsrand des abgeschrägten Endes (3) bestehen.

8. Prothese nach einem der Ansprüche 1 bis 7, **dadurch gekennzeichnet, dass** der röhrenförmige Körper (1) vorstehende äußere Ansätze (6) zum Halten der Prothese im Inneren des Kehlkopfes aufweist.

9. Prothese nach einem der Ansprüche 1 bis 8, **dadurch gekennzeichnet, dass** der röhrenförmige Körper (1) in Höhe der Stimmbänder einen Durchmesser aufweist, der geringer als derjenige ist, den er auf der Seite des mit einer Abschrägung gestalteten Endes (3) hat.
